# EUROPEAN PATENT APPLICATION

(11) **EP 2 135 872 A1**
(43) Date of publication of application: **23.12.2009**
(21) Application number: 08010975.4
(22) Date of filing: 17.06.2008
(51) Int. Cl.: C07D 519/04

(54) **Process for the preparation of bisindole alkaloid derivatives**

(71) Applicant: Indena S.p.A., 20139 Milano (IT)
(72) Inventor: Fontana, Gabriele, 20139 Milano (IT); Baldelli, Eleonora, 20139 Milano (IT); Riva, Sergio, 10131 Milano (IT); Danieli, Bruno, 20133 Milano (IT)
(74) Representative: Zumstein, Angela

(57) **Abstract**

A process for the coupling of catharantine and vindoline catalyzed by Laccases is described. The coupling produces anhydrovinblastine and anhydrovinblastine eniminium, which can be transformed to vinblastine or vinorelbine.

## Description

### Background of the invention

The leaves of *Catharanthus roseus* contain a variety of bisindole alkaloids, among which vinblastine (VBL, **1**) can be isolated after tedious and accurate chromatographic separations. VBL has been shown to be an important antitumor compound for the treatment of various carcinoma and, even today, is in great use in clinics. However, the low occurrence of **1** in plants and its troublesome isolation procedure make it difficult to provide this pharmacological agent in acceptable yields and at low cost. Thus, the synthesis of VBL was, and still remains, an important target in organic chemistry.

Total syntheses of VBL were developed by different groups, but they can be considered more a chemical exercise than a practical approach, because of the excessive number of synthetic steps and the consequently low isolated yields. Conversely, only few research groups have faced its semi-synthesis from more available natural precursors. All the reported approaches to VBL rely on the apparently simple formal addition of water to the C_{15'}-C_{20'} double bond of the recognized biosynthetic precursor anhydrovinblastine (AVBL, **2**, Scheme 1, path A).^{[1]} This synthetic goal was achieved via a multi-step sequence based on the oxidation of **2** to an iminium intermediate (7), followed by a 1,4-selective hydrogenation to form an enamine, which, in turn, was subjected to a sequential oxidation/reduction process to give VBL. Formation of 1 still occurred in low to poor yields and was inevitably accompanied by side products, among which the C-20' isomer of VBL (leurosidine) and other dimeric alkaloids were detected. In a series of papers, Kutney and coworkers described a "bio"-approach for the transformation of AVBL into VBL, exploiting bioconversions catalyzed by whole *Catharanthus roseus* cell cultures or by their crude enzymes cocktail (free or immobilized).^{[2]} Very recently, an innovative synthesis has been reported (16 % yield of **1** from **2**), which alternates oxidation and reduction steps, with oxygen and NaBH₄ respectively, in the presence of an anti-VBL monoclonal antibody acting as a chiral mould.^{[3]}

In turn, the recognized central role of AVBL in the formation of VBL, as well as of the semi- synthetic antitumoral alkaloid vinorelbine (3, Scheme 1, path B) together with its low natural availability, has spurred many efforts towards its synthesis. The most efficient approach to **2** is based on the emulation of the biosynthetic pathway, and consists in the condensation of the monomeric alkaloids catharanthine (**4**) and vindoline (**5**),^{[4]} both occurring as more frequent constituents in plants. In this biomimetic way, **4** is oxidized to generate an electrophilic species (**6**) that is captured by the nucleophile **5** to give a dimeric cationic intermediate **7** that, after NaBH₄ reduction, gives AVBL (Scheme 2). Different strategies have been tested to start the reactions sequence of Scheme 2, exploiting electrochemical, photochemical, chemical or enzymatic approaches.

The present invention now describes an efficient and environment friendly method for obtaining in large isolated amounts anhydrovinblastine **2** or its iminium derivative (**7**) as intermediates for the manufacturing of vinblastine or vinorelbin starting from naturally available precursors.

### Description of the invention

The inventors found that enzymes known as Laccases can efficiently activate catharantine for the oxidative coupling with vindoline to give anhydrovinblastine **2** or its iminium precursor 7.

Laccases are a group of oxidoreductases (also called "blue oxidases") widely distributed in nature. They are highly stable in solution, and catalyze the oxidation of several substrates under mild reaction conditions. The active site of these enzymes consists of a cluster of four copper atoms. All of them are involved in the cyclic redox radical mechanism in which one oxygen molecule is reduced to give two water molecules, while four substrate molecules are oxidized to the corresponding reactive radicals.^{[5]} Most of the reported applications are related to the oxidation of phenolic derivatives to give dimeric products or to the nuclear amination, domino or Diels-Alder reaction of *in situ* laccase-generated quinones.^{[6]}

The inventors of the present invention have used different laccases to catalyze the efficient and environmentally friendly oxidative coupling of the indolic alkaloids catharantine and vindoline using preferably air oxygen as the oxidant agent. Following borohybride reduction (preferably with NaBH₄) of the eniminium cationic intermediate, the synthetically useful dimer anhydrovinblastine (AVBL) can be isolated and characterized. Several suitable reaction parameters were tested and AVBL could be isolated in 56 % yields under optimized reaction conditions.

When catharanthine and vindoline are incubated with laccase according to the invention, after quenching preferably with NaBH₄, extraction preferably with CH₂Cl₂ and chromatographic purification, anhydrovinblastine (**2**) can be isolated in fairly good yields.

Several commercially available laccases (from *Trametes versicolor, Tv, Trametes pubescens, Tp; Rhus vernicifera, Rv; Pyricularia oryzae, Po; Agaricus bisporus, Ab; Mycelopthora termophila, Mt*) can be used for the oxidative coupling. Laccases from *Trametes* spp, *Agaricus bisporus* and *Mycelopthora termophila* are preferred.

In order both to recycle the enzyme and to be able to selectively remove the catalyst out of the reaction solution the Laccases are preferably immobilized on an inert carrier, preferably Eupergit© carrier or similar activated polymers like for example highly porous methacrylic polymers (e.g. Sepabeads©EC (Resindion srl, Binasco, Italy), Sepabeads©EC-EP or Sepabeads©EC-HFA), by covalent bonding. The immobilized laccases behave similarly to the free enzymes, and can be separated by simple filtration and reused for at least five reaction cycles without significant loss in activity.

A series of aqueous buffers having pH from 3.0 to 6.5, preferably acetate buffer at pH 4.5 can be used for the biotransformation, with a preference for 50 mM acetate buffer at pH 4.0.

The laccases used operate preferably in a range of temperature from 25 to 40 °C, with optimal results at 30°C preferably in the presence of air.

The biotransformation, according to the conditions, is completed in a period from 1 to 8 hours, preferably from 4 to 6 hours. In a typical embodiment of this invention the laccase has a concentration equal or higher than 4000 U per mmol of substrate.

The optional removal of the immobilized laccase is obtained by filtration. When the laccase is not removed, the substrate can be recovered by extraction with organic solvents, e.g. AcOEt or CH₂Cl₂, as described below in the examples.

The reduction of the eniminium cationic intermediate is carried out with hydrides, preferably, but not limited to sodium boro hydride (NaBH₄). The reaction is carried out in the same medium of the enzyme-catalyzed coupling, or in aqueous medium, and is generally completed in 1 hour. An efficient conversion is obtained when at least 4 equivalents of hydride are used.

The isolation of the products according to the present invention is generally carried out by extraction with organic solvents, e.g. AcOEt or CH₂Cl₂, from the aqueous media, and the purification is efficiently obtained by column chromatography.

As far as the production of anhydrovinblastine eniminium (7) is concerned the steps of oxidative coupling, removal of Laccase and isolation of the product can be carried out in the same way as described for the production of anhydrovinblastine.

Under the preferred reaction conditions, the laccase used couples vindoline and catharantine to give the eniminium **7** (95 % conversion of vindoline) and, following NaBH₄ treatment and silica chromatography of the crude residue, anhydrovinblastine is isolated in 56 % yields, as the examples of the present invention describe.
The following examples illustrate some embodiments of the present invention without limiting its scope.

### Examples

### A. Material and Methods

Catharantine (**4**) and vindoline (**5**) were purchased from Ajinomoto, Chematek SpA, Lainate, Italy. Laccase from *Trametes pubescens* was provided by Prof. Haltrich (BOKU University, Wien, Austria); laccases from *Trametes versicolor, Rhus vernicifera, Pyricularia oryzae, Agaricus bisporus* were from Sigma-Aldrich, Milano, Italy, whereas the enzyme from *Mycelopthora termophila* was from Novozymes. The other substrates and reagents were from Sigma-Aldrich. Eupergit C250L was from Rohm GmbH (Darmstadt, Germany).

Enzymatic activities were monitored using a Jasco V-530 UV/VIS spectrophotometer.

Thin-layer chromatography (TLC) was performed on silica plates (Merck 60 F₂₅₄). Substrates and products were visualized at 254 nm and/or by plates treatment with the CAS reagent ((NH₄)₂Ce(SO₄)₃.2H₂O, 1 g; 85 % H₃PO₄Ce(SO₄)₂, 100 ml).

Purifications were performed by flash chromatography on silica gel (Merck 60, 230-400 Mesh).
HPLC analyses were carried out using a Jasco 880-PU pump equipped with a Jasco 870-UV detector and a Hewlett-Packard HP-3395 integrator, using a C-18 RP column (LIChroCART^{®} 125-4, Aluspher^{®} 100, 5 µm) and a precolumn (LIChroCART^{®} 4-4, Aluspher^{®} 100, 5 µm). Eluent A: 5 mM phosphate buffer pH 6.5; eluent B: acetonitrile. Elution gradient: from eluent A - eluent B 80 : 20 to eluent A - eluent B 20 : 80 in 20 min; same eluent (eluent A - eluent B 20 : 80) for 20 min; flow rate 1 ml/min; UV detection at 294 nm. Retention time: Catharantine (**4**), 12.38 min; Vindoline (**5**), 5.87 min; anhydrovinblastine eniminium (**7**), 13.08 min; AVBL (**2**), 15,22. Alternatively it could be used a C-18 RP column (ZORBAX Eclipse XDB-C18, 4,6 x 150 mm, 5 µm, Agilent Technologies) and a precolumn (Eclipse XDB-C18, 4,6 x 12.5 mm, 5 µm, Agilent Technologies). Eluent A: 10 % (95 % acetonitrile and 5 % THF) plus 90 % 10 mM ammonium acetate buffer pH 6.5; eluent B: 80 % (95 % acetonitrile and 5 % THF) plus 20 % 10 mM ammonium acetate buffer pH 6.5. Elution gradient: from eluent A - eluent B 75 : 25 to eluent A - eluent B 0 : 100 in 30 min; same eluent (eluent A - eluent B 0 : 100) for 5 min; flow rate 1 ml/min; UV detection at 294 nm. Retention time: Catharantine (**4**), 16.17 min; Vindoline (**5**), 15.02 min; anhydrovinblastine eniminium (**7**), 11.05 min; AVBL (**2**), 21,10.

NMR spectra were recorded on a Bruker AC400 (400 MHz).

High resolution electrospraymass spectra (HRESI-MS) were acquired with an FT-ICR (Fourier Transfer Ion Cyclotron Resonance) APEXTM II model (Bruker Daltonics) equipped with a 4.7 Tesla cryo-magnet (Magnex). Samples were dissolved in CH₃CN and injected into the instrument equipped with its own ESI source. Spectra were recorded in the HR mode with resolutions ranging from 20000 to 30000.

### B. Spectrophotometric assay of laccase activity

Laccase activity was evaluated by monitoring the oxidation of ABTS at 436 nm, following a standard protocol. An enzymatic solution (10 µl) was added to a 1 ml cuvette containing 20 mM acetate buffer pH 3.5 (890 µl) and ABTS (100 µl of a 10 mM solution of ABTS in H₂O). One enzyme unit is defined as the amount of laccase that oxidizes 1 µmol of ABTS under this condition (λ_{ABTS} = 29.3 mM⁻¹ cm⁻¹).

### C. Laccase activity in the presence of organic cosolvents

The evaluation of the effect of a fixed concentration (10 %, 20 %, or 30 % v/v) of several water-miscible organic cosolvents (acetone, dioxane, acetonitrile, THF, DMF, DMSO, EtOH, MeOH, *i*-PrOH) on laccase activity was performed by spectrophotometric analysis using the previously described assay conditions. The total volume of solution in cuvette was 1 ml, made of 20 mM acetate buffer pH 3.5 (700-900 µL) and the respective cosolvent (100-300 µL). The residual activities were evaluated and compared to the data obtained in the absence of cosolvents.

### D. Immobilization of Trametes pubescens laccase on Eupergit C250L

A sample of the crude enzyme preparation (50 mg, 5.850 total U, specific activity 532 U/mg) was dissolved in 600 µL of 0.1 M phosphate buffer, pH 7.0. Additional phosphate buffer (3.1 mL, 1.17 M) was added to this solution, in order to get a resulting 1 M phosphate buffer, pH 7. A sample of this mother solution (50 µL) was stored below 0 °C to be used as a control.
The polymeric matrix carrying reactive epoxidic groups Eupergit C250L (750 mg) was added to 3.7 mL of this solution and the resulting slurry was stored overnight in the fridge, being mixed at regular interval. After 24 h, the slurry was centrifuged (3.000 rpm, 5 min) and washed 3 times with 5 mL of 0.1 phosphate buffer pH 7. The residual laccase activity due to the unbound enzyme was measured by spectrophotometric assay) and resulted to be 131 U tot (2.2 %). The immobilized enzyme was then re-suspended in a 5 mL solution of 0.3 M ethanolamine in phosphate buffer (1.2 M, pH 7.0) and stored in the fridge for 5 h, being mixed at regular intervals. The solution was centrifugated (3000 rpm, 5 min) and washed 3 times with 5 mL of 0.1 M phosphate buffer pH 7.0. The immobilized enzyme was stored at 4 °C in a 10 mL acetate buffer (50 mM, pH 4.0).

### Laccase-catalyzed coupling of catharantine (4) and vindoline (5): preferred embodiment for step a.

The best results are being obtained using a *Trametes* laccase in 50 mM acetate buffer, pH 4.0, mildly shaken (120 rpm) at 30 °C under oxygenating conditions (1:1 v/v air-water biphasic system or continuous aereation) and without organic cosolvents.

### Example 1

### Production of anhydrovinblastine (2)

Catharanthine (440 mg, 1.31 mmol, 1.36 eq.) was suspended in 50 mM acetate buffer (pH 3.5, 41 ml) and vigorously shaken for 10 minutes at 45 °C in order to dissolve it completely. Vindoline (440 mg, 0.96 mmol, 1 eq.) was then added and the reaction solution was shaken for additional 10 minutes (vindoline was not completely dissolved). The solution pH was adjusted to 4.0 by adding 1 M AcOH. *Trametes versicolor* laccase (170 mg, 4250 U) was dissolved in 50 mM acetate buffer (pH 4.0, 9 ml) and the solution was dropped into the reaction mixture, which was gently shaken (120 rpm) at 30 °C (vindoline was completely dissolved after 1 h) and monitored by TLC and HPLC

After 8 h, HPLC analysis revealed the presence of 4 % residual vindoline. The solution was filtered to separate the denaturated protein and the pH was adjusted to 7.0 by adding 1 M NaOH. NaBH₄ was added (41 mg) and the resulting solution extracted with AcOEt (3 x 50 ml). The organic solvent was, dried over Na₂SO₄ and evaporated under reduced pressure. The crude residue was purified by silica gel chromatography (eluent: *tert*-butyl methyl ether - methanol - TEA 85 : 15 to eluate the residual starting materials; tert butyl methyl ether - methanol - TEA 85 : 15 : 0.5 to eluate anhydrovinblastine ) to give 415 mg of anhydrovinblastine (56 % isolated yield).

AVBL (**2**) ¹H-NMR (*d₆*-acetone): 7.48 (1 H, d, *J =* 7.7 Hz, H-9'), 7.39 (1 H , d, J= 8.0 Hz, H-12'), 7.05 (1 H, t, *J* = 7.8 Hz, H-11'), 7.00 (1 H, t, J = 7.8 Hz, H-10'), 6.81 (1 H, s, H-9), 6.37 (1 H, s, H-12), 5.80 (1 H, dt, *J₁* = 8.0 Hz, *J₂* = 11.0 Hz, H-14), 5.41 (1 H, d, J = 6 Hz, H-15'), 5.26 (1 H, d, *J₁* = 9.0 Hz, H-15), 3.84 (3 H, s, OMe), 3.70 (3 H, s, COOMe (vind)), 3.62 (1 H, s, H-2), 3.45 (3 H, s, COOMe (cat)), 3.48 (1 H, H-21'b), 3.40 (2 H, d, H-3), 3.34 (1 H, H-21'a), 3.20 (H-5b, H-5'b), 2.75 (1 H, s, H-21), 2.75 (1 H, m, H-5'a), 2.73 (3 H, s, NMe), 2.55 (2 H, d, J = 10.0 Hz, H-3'), 2.40 (3 H, m, H-17', H 5a), 2.15 (1 H, m, H-6a), 1.95 (3 H, s, OCOCH₃), 1.90 (2 H, m, H-19'), 1.88 (1 H, m, H-6b), 1.60 (1 H, dq, *J₁ =* 13.9, *J₂ =* 6.9, H-19b), 1.38 (1 H, dq, *J₁* = 7.0, *J₂* = 14.0, H-19a), 0.98 (3 H, t, J= 7.3, H-18'), 0.75 (3 H, t, H-18).

¹³C-NMR (*d₆*-acetone): 7.83 (C-18), 11.94 (C-18'), 20.20 (*CH₃*-CO-O), 27.86 (C-19'), 31.21 (C-6'), 31.65 (C-19), 33.57 (C-14'), 34.96 (C-17'), 37.38 (N- *CH₃*), 42.77 (C-6), 43.25 (C-20), 44.86 (C-3), 46.37 (C-3'), 49.94 (C-5), 50.78 (C-5'), 50.78 (COO*CH₃*), 50.88 (COO*CH₃*), 52,52 (C-21'), 52.84 (C-7), 54.25 (C-16), 55.54 (ArO*CH₃*), 65.23 (C-21), 76.30 (C-17), 79.63 (C-16), 83.30 (C-2), 94.09 (C-12), 116.12 (C-7'), 118.48 (C-9'), 118.69 (C-10'), 121.03 (C-8), 121.07 (C-10), 123.50 (C-11'), 124.04 (C- 9), 124.17 (C-15'), 124.32 (C-14), 129.37 (C-8'), 131.52 (C-15), 131.71 (C-2'), 135.58 (C-13'), 140.44 (C-20'), 153.06 (C-13), 158.23 (C-11), 170.06 (*CO*OCH₃), 171.52 (OCOCH₃), 174.27(*CO*OCH₃).

ESI-MS, positive mode: 793,41492 [M + H]⁺ (theoretic for : C₄₆H₅₇N₄O₈, 793.41709). CD: λ max 258 nm, Δε = +18.4; λ max 305 nm, Δε = + 8.2 (lit: λ max 258 nm, Δε = +14.0; λ max 305 nm, Δε = + 6.5)

### Example 2

### Production of AVBL (2) by the coupling catalyzed by the immobilized Trametes pubescens laccase obtained according to item D

Catharantine (**4**, 200 mg) and vindoline (**5**, 200 mg) were added to 50 mM acetate pH 3,5 (16 mL). The pH value was adjusted to 4.0 with glacial AcOH, the immobilized enzymatic suspension (4 mL, 2.200 U) was added and the reaction was mildly shaken (120 rpm) at 30 °C for 3.5 h (monitored by TLC and RP-HPLC). The enzyme was filtered and washed with acetate buffer. The reaction products were reduced by adding NaBH₄ (16 mg) and extracted with AcOEt (150 ml). The solvent was evaporated and the crude residue purified by silica gel flash chromatography to give 197 mg of AVBL (2), that was subsequently crystallized from cold methanol. Following this protocol, the immobilized biocatalyst was reused for 5 consecutive cycles.

### Example 3

### Production of anhydrovinblastine eniminium (7)

Catharanthine (110 mg, 0.33 mmol, 1.36 eq.) was suspended in 50 mM acetate buffer (pH 3.5, 10 ml) and vigorously shaken for 10 minutes at 45 °C in order to dissolve it completely. Vindoline (110 mg, 0.24 mmol, 1 eq.) was then added and the reaction solution was shaken for additional 10 minutes (vindoline was not completely dissolved). The solution pH was adjusted to 4.0 by adding 1 M AcOH. *Trametes versicolor* laccase (40 mg, 1000 U) was dissolved in 50 mM acetate buffer (pH 4.0, 3 ml) and the solution was dropped into the reaction mixture, which was gently shaken (120 rpm) at 30 °C and monitored by TLC and HPLC. After 8 h the solution was filtered to separate the denaturated protein, frozen and lyophilized. The residue was purified by preparative chromatography on C18 column.

HPLC analyses were carried out using a Jasco 880-PU pump equipped with a Jasco 870-UV detector and a Hewlett-Packard HP-3395 integrator, using a C-18 RP column (LIChroCART^{®} 125-4, Aluspher^{®} 100, 5 µm) Eluent A: 5 mM phosphate buffer pH 6.5; eluent B: acetonitrile. Elution gradient: from eluent A - eluent B 80 : 20 to eluent A - eluent B 20 : 80 in 20 min; same eluent (eluent A - eluent B 20 : 80) for 20 min; flow rate 1 ml/min; UV detection at 294 nm. Retention time: Catharanthine (**4**), 12.38 min; Vindoline (**5**), 5.87 min; anhydrovinblastine eniminium (**7**), 13.08 min; AVBL (2), 15,22.

### Legends to Schemes

Scheme 1. Synthesis of vinblastine (**1**) and vinorelbine (**3**) from chataranthine (**4**) and vindoline (**5**) *via* anhydrovinblastine (**2**).
Scheme 2. Synthetic sequence to produce anhydrovinblastine (**2**) from catharantine (**4**) and vindoline (**5**).

### References

(1) Scott, A. I.; Gueritte, F.; Lee, S. L. J. Am. Chem. Soc. 1978, 100, 6253-6255.
(2) (a) Kutney, J. P.; Aweryn, B.; Choi, L. S. L.; Kolodziejczyk, P.; Kurz, W. G. W.; Chatson, K.; Constabel, F. Helv. Chim. Acta 1982, 65, 1271-1278. (b) Kutney, J. P. Nat. Prod. Rep. 1990, 6, 85-103, and references therein.
(3) Shirahama, T.; Kohno, T.; Kaijima, T.; Nagaoka, Y.; Morimoto, D.; Hirata, K.; Uesato, S. Chem. Pharm. Bull. 2006, 54, 665-668.
(4) For a review, see: Sundberg, R. J.; Smith, S. Q. The Iboga Alkaloids and their Role as Precursors of Anti-Neoplastic Bisindole Catharanthus Alkaloids. In The Alkaloids, Chemistry and Biology; Cordell, G. A. Ed.; Academic Press Inc.; San Diego, CA, 2002; Vol. 59, pp 281-372.
(5) For recent reviews see: (a) Riva, S. Trends Biotechnol. 2006, 24, 219-226. (c) Claus, H. Micron 2004, 35, 93-96. (d) Burton, S. G. Curr. Org. Chem. 2003, 7, 1317-1332. (e) Mayer, M. M.; Staples, R. C. Phytochemistry 2002, 60, 551-565.
(6) See, for instance: (a) Ncanana, S.; Baratto, L.; Roncaglia, S.; Riva, S.; Gale Burton, S. Adv. Synth. Catal. 2007, 349, 1507-1513. (b) Ponzoni, C.; Benedenti, E.; Cramarossa, M. R.; Raimondi, S.; Travisi, G.; Pagnoni, U. M.; Riva, S.; Forti, L. Adv. Synth. Catal. 2007, 349, 1497-1506. (c) Witayakran, S.; Zettili, A.; Ragauskas, A. J. Tetrahedron Lett. 2007, 48, 2983-2987. (d) Hajdok, S.; Leutbecher, H.; Greiner, G.; Conrad, J.; Beifuss, U. Tetrahedron Lett. 2007, 48, 5073-5076. (e) Mikolasch, A.; Niedermeyer, T. H. J.; Lalk, M.; Witt, S.; Seefeldt, S.; Hammer, E.; Schauer, F.; Salazar, M. G.; Hessel, S.; Julich, W. D.; Lindequist, U. Chem. Pharm. Bull. 2007, 55, 412-416. (f) Niedermeyer, T. H. J.; Lalk, M. J. Mol. Cat. B-Enzymatic 2007, 45, 113-117.

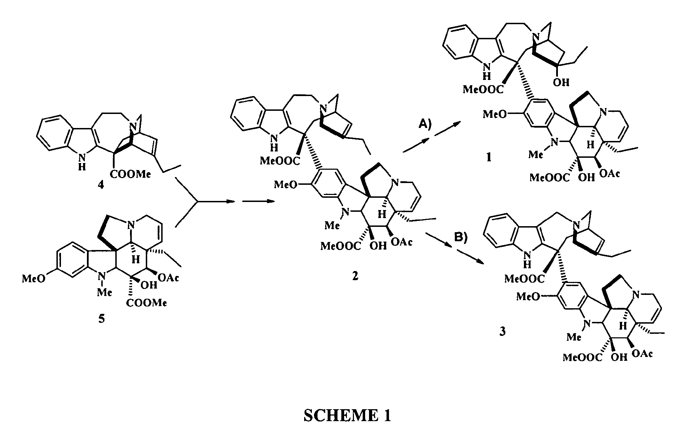

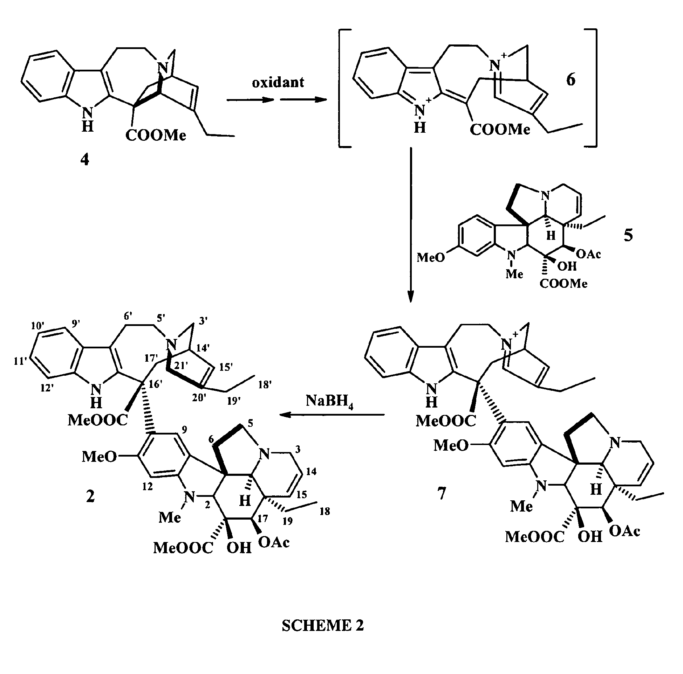

## Claims

1. Process for producing anhydrovinblastine by coupling vindoline and catharantine comprising the steps
a. oxidative coupling catalyzed by Laccases
b. optional removal of laccase
c. reduction with borohydrides
d. isolation of anhydrovinblastine

2. Process for producing anhydrovinblastine eniminium (7) by coupling vindoline and catharantine comprising the steps
a. oxidative coupling catalyzed by Laccases
b. optional removal of laccase
c. isolation of anhydrovinblastine eniminium

3. Process according to claim 1 or claim 2 wherein the oxidative coupling is performed in aqueous buffer having pH from 3.0 to 6.5, preferably from 4.0 to 4.5.

4. Process according to claim 3 wherein the aqueous buffer is a acetate buffer.

5. Process according to claim 4 wherein the aqueous buffer is a 50 mM acetate buffer.

6. Process according to claim 1 or claim 2 wherein the oxidative coupling is performed at a temperature in the range from 25 to 40°C.

7. Process according to claim 6 wherein the oxidative coupling is performed at 30°C.

8. Process according to claim 1 or claim 2 wherein the laccase stems from the group consisting of *Trametes pubescens, Trametes versicolor, Trametes spp, Agaricus bisporus* and *Mycelopthora termophila* and mixtures thereof.

9. Process according to claim 8 wherein the laccase stems from *Trametes spp.*

10. Process according to claim 8 wherein the laccase stems from *Agaricus bisporus.*

11. Process according to claim 8 wherein the laccase stems from *Mycelopthora termophila.*

12. Process according to claim 1 or claim 2 wherein the laccase is immobilized on an inert carrier.

13. Process according to claim 12 wherein the inert carrier is selected form the group consisting of Eupergit, Sepabeads EC, Sepabeads EC-EP, Sepabeads EC-HFA and mixtures thereof.

14. Process according to claim 13 wherein the inert carrier is Eupergit.
